# EUROPEAN PATENT APPLICATION

(11) **EP 1 801 221 A1**
(43) Date of publication of application: **27.06.2007**
(21) Application number: 05028139.3
(22) Date of filing: 22.12.2005
(51) Int. Cl.: C12N 15/79, C12P 1/02

(54) **Promoter sequences**

(71) Applicant: SANDOZ AG, 4056 Basel (CH)
(72) Inventor: Eichhorn, Heiko, 6250 Kundl (AT); Specht, Thomas, 6250 Kundl (AT); Zadra, Ivo, 83052 Heufeld (DE)
(74) Representative: Dietz, Jörg-Reimar

(57) **Abstract**

The present invention relates to methods for producing polypeptides in filamentous fungi. The present invention also relates to isolated *Penicillium* promoter sequences and to nucleic acid constructs, vectors, and host cells comprising the promoter sequences operably linked to nucleic acid sequences encoding polypeptides.

## Description

The present invention relates to methods for producing polypeptides in filamentous fungi. The present invention also relates to isolated *Penicillium* promoter sequences and to nucleic acid constructs, vectors, and host cells comprising the promoter sequences operably linked to nucleic acid sequences encoding polypeptides.

The recombinant production of homologous and heterologous proteins in a fungal host cell, particularly a filamentous fungal cell such as *Penicillium* allows production of proteins in commercially relevant quantities.
Recombinant production of a protein is accomplished by constructing an expression vector in which the DNA coding for the protein is placed under the expression control of a promoter, excised from a regulated gene, suitable for the host cell. The expression vector is introduced into the host cell, usually by plasmid-mediated transformation. Production of the protein is then achieved by culturing the transformed host cell under suitable conditions necessary for the proper functioning of the promoter contained in the expression vector.

The filamentous fungi *Penicillium chrysogenum* and *Acremonium chrysogenum* have the ability to produce antibiotics like penicillin and cephalosporin and are therefore of industrial interest. In order to increase productivity of commercially used production strains techniques for genetic manipulation of these strains have been developed in the last couple of years. These techniques include the transformation of protoplasts with vectors including a selection marker like the phleomycin resistance gene (Kolar, M. et al. (1988), Gene 62, 127-134). The expression of genes of interest and the replacement of the promoters of these genes by other homologous or heterologous promoters in order to improve gene expression has been shown. Especially for the expression of negatively regulated genes promoters of constitutively highly expressed genes have been identified which do not show negative catabolic regulation, hereinafter called high constitutive promoters.
The inactivation of gene expression, either partially or almost completely, is used to eliminate or at least down-regulate activities of enzymes or regulators in order to eliminate undesirable regulatory pathways or regulators in a specific signaling cascade. In most cases gene disruption is not appropriate because of the level of ploidy or because it results in a non-viable organism.

The use of antisense constructs to modulate gene regulation or blocking enzyme activities has been shown in *Penicillium chrysogenum, Aspergillus nidulans* and several other filamentous fungi as a valuable approach, in particularly if loss-of-function mutations exhibit impaired growth parameters making this strategy inapplicable for industrial purposes (Zadra et al. (2000), Appl. Environ. Microbiol. 66, 4810-4816; Bautista, L. F. et al. (2000), Appl. Environ. Microbiol. 66(10), 4579-4581 and Wang, T. H. et al. (2005), Lett. Appl. Microbiol. 40, 424-429.)
In addition to the well known antisense technology, RNA-mediated gene silencing, a new method to alter gene expression, is extensively characterized in various model organisms from plants to animals. Recently this technology is also established in filamentous fungi.
The RNA-mediated gene silencing is a posttranscriptional gene-silencing in which doublestranded RNA (dsRNA) triggers the degradation of cognate mRNA in a sequence-specific manner (Nakayashiki, H. et al. (2005), Fungal Genetics and Biology 42, 275-283).
Despite the proof that RNA-mediated gene silencing is functional in several fungal species (Nakayashiki, H. et al. (2005), Fungal Genetics and Biology 42, 275-283; Kadotani, N. et al. (2003) Mol. Plant-Microbiol. Interactions 16, 769-776 and Mouyna, I. et al. (2004), FEMS Microbiol. Lett. 237, 317-324) for filamentous fungi this technology lies only in the beginnings. This is mainly due to limited genetic tools available.

Therefore, there is a need in the art for new promoters for controlling gene expression in filamentous fungi.

The present invention provides improved methods for producing a polypeptide in a fungal host cell and new promoters for such production.

### Summary of the Invention

The present invention relates to a method for producing a polypeptide, comprising the steps of:
(a) cultivating a fungal host cell in a medium conductive for the production of the polypeptide, wherein the fungal host cell comprises a first nucleic acid sequence encoding the polypeptide operably linked to a second nucleic acid sequence comprising a promoter sequence selected from the group consisting of SEQ ID NO. 1, SEQ ID NO. 2, SEQ ID NO. 3, SEQ ID NO. 4, SEQ ID NO. 5, and subsequences thereof, and mutant, hybrid, and tandem promoters thereof; and
(b) isolating the polypeptide from the culture medium.

The present invention also relates to isolated nucleic acid sequences comprising a nucleic acid sequence selected from the group consisting of SEQ ID NO. 1, SEQ ID NO. 2, SEQ ID NO. 3, SEQ ID NO. 4, SEQ ID NO. 5 and subsequences thereof.

The present invention also relates to isolated nucleic acid sequences, selected from the group consisting of
(a) a nucleic acid sequence having at least 80% homology with a sequence selected from the group consisting of SEQ ID NO. 1, SEQ ID NO. 2, SEQ ID NO. 3, SEQ ID NO. 4 and SEQ ID NO. 5;
(b) a nucleic acid sequence which hybridizes under conditions of stringency with (i) a sequence selected from the group consisting of SEQ ID NO. 1, SEQ ID NO. 2, SEQ ID NO. 3, SEQ ID NO. 4 and SEQ ID NO. 5; (ii) a subsequence of (i) of at least 100 nucleotides, or (iii) a complementary strand of (i) or (ii);
(c) a nucleic acid sequence comprising a substitution, deletion, and/or insertion of one or more nucleotides;
(d) an allelic variant of (a), (b), or (c); and
(e) a subsequence of (a), (b), (c), or (d).

The present invention also relates to nucleic acid constructs comprising a nucleic acid sequence encoding a polypeptide operably linked to the nucleic acid sequence described above. It further relates to recombinant expression vectors and a recombinant host cells comprising the nucleic acid constructs.

### Brief Description of the Figures

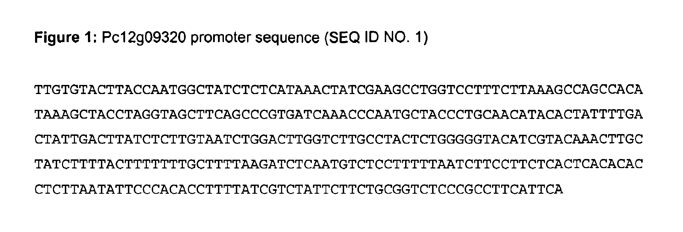

Figure 1 shows the Pc12g09320 promoter sequence (SEQ ID NO. 1)

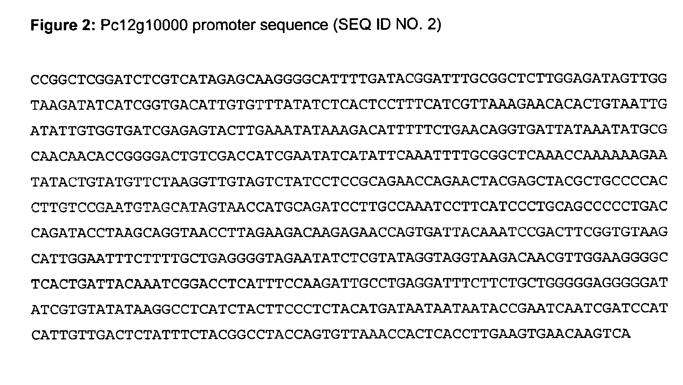

Figure 2 shows the Pc12g10000 promoter sequence (SEQ ID NO. 2)

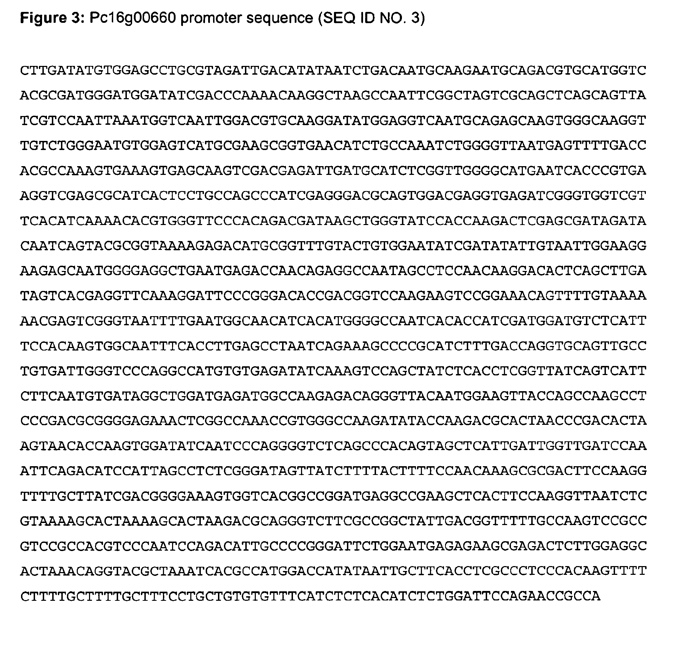

Figure 3 shows the Pc16g00660 promoter sequence (SEQ ID NO. 3)

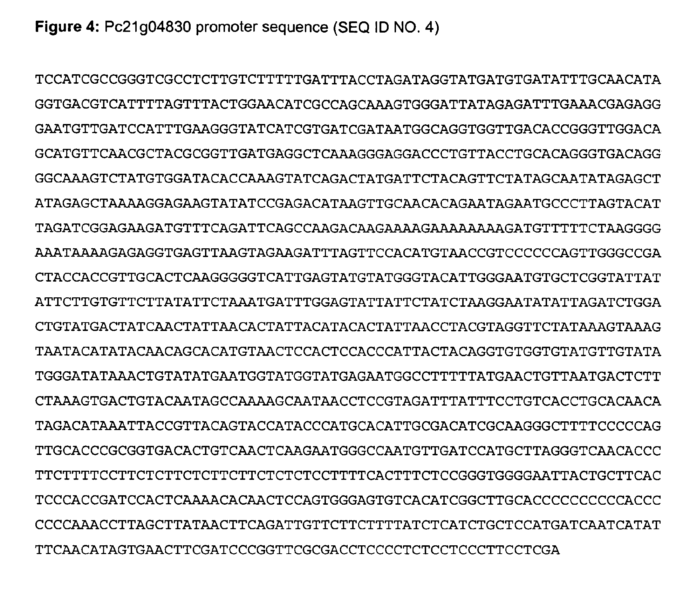

Figure 4 shows the Pc21 g04830 promoter sequence (SEQ ID NO. 4)

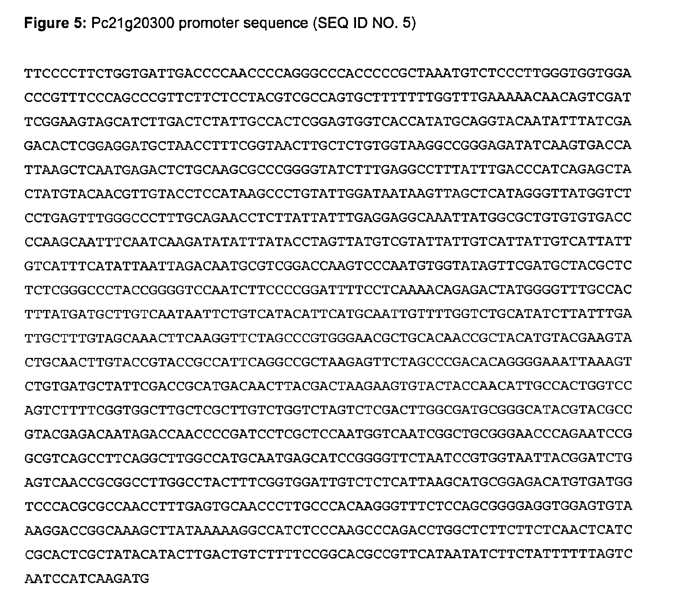

Figure 5 shows the Pc21 g20300 promoter sequence (SEQ ID NO. 5)

### Detailed Description of the Invention

The present invention relates to a method for producing a polypeptide, comprising the steps of:
(a) cultivating a fungal host cell in a medium conductive for the production of the polypeptide, wherein the fungal host cell comprises a first nucleic acid sequence encoding the polypeptide operably linked to a second nucleic acid sequence comprising a promoter sequence selected from the group consisting of SEQ ID NO. 1, SEQ ID NO. 2, SEQ ID NO. 3, SEQ ID NO. 4, SEQ ID NO. 5, and subsequences thereof, and mutant, hybrid, and tandem promoters thereof; and
(b) isolating the polypeptide from the culture medium.

Preferably, the fungal host cell in step a) is a filamentous fungal host cell. More preferably, the fungal host cell in step a) is Penicillium chrysogenum or Acremonium chrysogenum or Aspergillus terreus or Penicillium citrinum.

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.
The resulting polypeptide may be recovered by methods known in the art. For example, the polypeptide may be recovered from the nutrient medium by conventional procedures including, but not limited to, centrifugation, filtration, extraction, spray- drying, evaporation, or precipitation.
The polypeptides may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS- PAGE, or extraction (see, e.g., Protein Purification, J.-C. Janson and Lars Ryden, editors, VCH Publishers, New York, 1989).

The term "promoter" is defined herein as a DNA sequence that binds RNA polymerase and directs the polymerase to the correct downstream transcriptional start site of a nucleic acid sequence encoding a polypeptide to initiate transcription. RNA polymerase effectively catalyzes the assembly of messenger RNA complementary to the appropriate DNA strand of the coding region. The term "promoter" will also be understood to include the 5' non-coding region (between promoter and translation start) for translation after transcription into mRNA, cis-acting transcription control elements such as enhancers, and other nucleotide sequences capable of interacting with transcription factors.
The term "mutant promoter" is defined herein as a promoter having a nucleotide sequence comprising a substitution, deletion, and/or insertion of one or more nucleotides of a parent promoter, wherein the mutant promoter has more or less promoter activity than the corresponding parent promoter. The term "mutant promoter" will also encompass natural variants and in vitro generated variants obtained using methods well known in the art such as classical mutagenesis, site-directed mutagenesis, and DNA shuffling.
The term "hybrid promoter" is defined herein as parts of two or more promoters that are fused together to generate a sequence that is a fusion of the two or more promoters, which is operably linked to a coding sequence and mediates the transcription of the coding sequence into mRNA.
The term "tandem promoter" is defined herein as two or more promoter sequences each of which is operably linked to a coding sequence and mediates the transcription of the coding sequence into mRNA.
The term "operably linked" is defined herein as a configuration in which a control sequence, e.g., a promoter sequence, is appropriately placed at a position relative to a coding sequence such that the control sequence directs the production of a polypeptide encoded by the coding sequence.
The term "coding sequence" is defined herein as a nucleic acid sequence that is transcribed into mRNA which is translated into a polypeptide when placed under the control of the appropriate control sequences. The boundaries of the coding sequence are generally determined by the ATG start codon located just upstream of the open reading frame at the 5' end of the mRNA and a transcription terminator sequence located just downstream of the open reading frame at the 3' end of the mRNA. A coding sequence can include, but is not limited to, genomic DNA, cDNA, semisynthetic, synthetic, and recombinant nucleic acid sequences.
In a preferred embodiment of the method for producing a polypeptide above, the promoter has the nucleic acid sequence of SEQ ID NO. 1 or a subsequence thereof. In another preferred embodiment, the promoter has the nucleic acid sequence of SEQ ID NO. 2 or a subsequence thereof.
In another preferred embodiment, the promoter has the nucleic acid sequence of SEQ ID NO. 3 or a subsequence thereof.
In another preferred embodiment, the promoter has the nucleic acid sequence of SEQ ID NO. 4 or a subsequence thereof.
In another preferred embodiment, the promoter has the nucleic acid sequence of SEQ ID NO. 5 or a subsequence thereof.

In the methods of the present invention, the promoter may also be a mutant of the promoters described above having a substitution, deletion, and/or insertion of one or more nucleotides.
A mutant promoter may have one or more mutations. Each mutation is an independent substitution, deletion, and/or insertion of a nucleotide. The introduction of a substitution, deletion, and/or insertion of a nucleotide into the promoter may be accomplished using any of the methods known in the art such as classical mutagenesis, site-directed mutagenesis, or DNA shuffling.

In the methods of the present invention, the promoter may also be a hybrid promoter comprising a portion of one or more promoters of the present invention; a portion of a promoter of the present invention and a portion of another promoter, e.g., a leader sequence of one promoter and the transcription start site from the other promoter; or a portion of one or more promoters of the present invention and a portion of one or more other promoters.
The other promoter may be any promoter sequence which shows transcriptional activity in the fungal host cell of choice including a mutant, truncated, and hybrid promoter, and may be obtained from genes encoding extracellular or intracellular polypeptides either homologous or heterologous to the host cell. The other promoter sequence may be native or foreign to the nucleic acid sequence encoding the polypeptide and native or foreign to the cell.

Examples of other promoters useful in the construction of hybrid promoters with the promoters of the present invention include the promoters obtained from the genes which are regulated, for example induced or repressed by any compounds of the culture medium, like carbohydrates, nitrogen, phosphate, sulphur etc. or any chemical inductor known in the art. Promoters useful in the construction of hybrid promoters with the promoters of the present invention may also be induced or repressed by any physical condition, for example temperature.

The promoter may also be a tandem promoter comprising two more promoters of the present invention or alternatively one or more promoters of the present invention and one or more other promoters, such as those exemplified above. The two or more promoter sequences of the tandem promoter may simultaneously promote the transcription of the nucleic acid sequence. Alternatively, one or more of the promoter sequences of the tandem promoter may promote the transcription of the nucleic acid sequence at different stages of growth of the cell.

A mutant, hybrid, or tandem promoter of the present invention has at least about 20%, preferably at least about 40%, more preferably at least about 60%, more preferably at least about 80%, more preferably at least about 90%, more preferably at least about 100%, even more preferably at least about 200%, most preferably at least about 300%, and even most preferably at least about 400% of the promoter activity of the promoter of SEQ ID NO. 1, SEQ ID NO. 2, SEQ ID NO. 3, SEQ ID NO. 4 or SEQ ID NO. 5.

The polypeptide encoded by the nucleic acid sequence may be native or heterologous to the fungal host cell of interest.
The term "polypeptide" is not meant herein to refer to a specific length of the encoded product and, therefore, encompasses peptides, oligopeptides, and proteins. The term "heterologous polypeptide" is defined herein as a polypeptide which is not native to the fungal cell, a native polypeptide in which modifications have been made to alter the native sequence, or a native polypeptide whose expression is quantitatively altered as a result of a manipulation of the fungal cell by recombinant DNA techniques. For example, a native polypeptide may be recombinantly produced by, e.g., placing a gene encoding the polypeptide under the control of a promoter of the present invention to enhance expression of the polypeptide, to expedite export of a native polypeptide of interest outside the cell by use of a signal sequence, and to increase the copy number of a gene encoding the polypeptide normally produced by the cell. The fungal cell may contain one or more copies of the nucleic acid sequence encoding the polypeptide. The native or homologue polypeptide as well as the heterologous polypeptide may be either a non-secreted or a secreted polypeptide.

Preferably, the polypeptide is a native or homologue polypeptide. In a preferred embodiment, the polypeptide is a non-secreted polypeptide.

The present invention also relates to an isolated nucleic acid sequence comprising a nucleic acid sequence selected from the group consisting of SEQ ID NO. 1, SEQ ID NO. 2, SEQ ID NO. 3, SEQ ID NO. 4, SEQ ID NO. 5 and subsequences thereof.

In a preferred embodiment, the isolated nucleic acid sequence comprises the nucleic acid sequence of SEQ ID NO. 1 or a subsequence thereof.
In another preferred embodiment, the isolated nucleic acid sequence comprises the nucleic acid sequence of SEQ ID NO. 2 or a subsequence thereof.
In another preferred embodiment, the isolated nucleic acid sequence comprises the nucleic acid sequence of SEQ ID NO. 3 or a subsequence thereof.
In another preferred embodiment, the isolated nucleic acid sequence comprises the nucleic acid sequence of SEQ ID NO. 4 or a subsequence thereof.
In another preferred embodiment, the isolated nucleic acid sequence comprises the nucleic acid sequence of SEQ ID NO. 5 or a subsequence thereof.

The present invention also relates to nucleic acid constructs comprising a nucleic acid sequence encoding a polypeptide operably linked to a promoter of the present invention and one or more control sequences which direct the expression of the coding sequence in a suitable host cell under conditions compatible with the control sequences.
The term nucleic acid construct is synonymous with the term expression cassette when the nucleic acid construct contains a coding sequence and all the control sequences required for expression of the coding sequence.
In the methods of the present invention, the nucleic acid sequence may comprise one or more native control sequences or one or more of the native control sequences may be replaced with one or more control sequences foreign to the nucleic acid sequence for improving expression of the coding sequence in a host cell.
The term "control sequences" is defined herein to include all components which are necessary or advantageous for the expression of a polypeptide of the present invention.
Each control sequence may be native or foreign to the nucleic acid sequence encoding the polypeptide. Such control sequences include, but are not limited to, a leader, polyadenylation sequence, propeptide sequence, promoter of the present invention, signal peptide sequence, and transcription terminator. At a minimum, the control sequences include a promoter of the present invention, and transcriptional and translational stop signals. The control sequences may be provided with linkers for the purpose of introducing specific restriction sites facilitating ligation of the control sequences with the coding region of the nucleic acid sequence encoding a polypeptide.

The present invention also relates to recombinant expression vectors comprising a promoter of the present invention, a nucleic acid sequence encoding a polypeptide, and transcriptional and translational stop signals. The various nucleic acid and control sequences described above may be joined together to produce a recombinant expression vector which may include one or more convenient restriction sites to allow for insertion or substitution of the promoter and/or nucleic acid sequence encoding the polypeptide at such sites. Alternatively, the nucleic acid sequence may be expressed by inserting the nucleic acid sequence or a nucleic acid construct comprising the promoter and/or sequence into an appropriate vector for expression. In creating the expression vector, the coding sequence is located in the vector so that the coding sequence is operably linked with a promoter of the present invention and one or more appropriate control sequences for expression.
The recombinant expression vector may be any vector (e.g., a plasmid or virus) which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids.
The vector may be an autonomously replicating vector, i.e., a vector which exists as an extrachromosomal entity, the replication of which is independent of chromosomal replication, e.g., a plasmid, an extrachromosomal element, a minichromosome, or an artificial chromosome. The vector may contain any means for assuring self-replication.

Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used. The vector may be introduced into the host cell in combination with one or more additional vectors by the method of co-transformation.
The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Suitable markers for use in a filamentous fungal host cell include, but are not limited to, hygromycin or phleomycin. Further suitable markers are for example described in Finklstein, Ball (eds.), Applied Molecular Genetics of Filamentous fungi (ibid.) Biotechnology of Filamentous fungi, Butterworth-Heinemann, Boston, 1992.

The present invention also relates to recombinant host cells, comprising a promoter of the present invention operably linked to a nucleic acid sequence encoding a polypeptide, which are advantageously used in the recombinant production of the polypeptides. A vector comprising a promoter of the present invention operably linked to a nucleic acid sequence encoding a polypeptide is introduced into a host cell so that the vector is maintained as a chromosomal integrant or as a self- replicating extra- chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.
The host cell may be any fungal cell useful in the methods of the present invention. In a preferred embodiment, the fungal host cell is a filamentous fungal cell. Preferably, the filamentous fungal cell is Penicillium chrysogenum or Acremonium chrysogenum or Aspergillus terreus or Penicillium citrinum.

The present invention also relates to isolated nucleic acid sequences, selected from the group consisting of
(a) a nucleic acid sequence having at least 80% homology with a sequence selected from the group consisting of SEQ ID NO. 1, SEQ ID NO. 2, SEQ ID NO. 3, SEQ ID NO. 4 and SEQ ID NO. 5;
(b) a nucleic acid sequence which hybridizes under conditions of stringency with (i) a sequence selected from the group consisting of SEQ ID NO. 1, SEQ ID NO. 2, SEQ ID NO. 3, SEQ ID NO. 4 and SEQ ID NO. 5; (ii) a subsequence of (i) of at least 100 nucleotides, or (iii) a complementary strand of (i) or (ii);
(c) a nucleic acid sequence comprising a substitution, deletion, and/or insertion of one or more nucleotides;
(d) an allelic variant of (a), (b), or (c); and
(e) a subsequence of (a), (b), (c), or (d).

In one embodiment, the present invention relates to isolated nucleic acid sequences having at least 80% homology with a sequence selected from the group consisting of SEQ ID NO. 1, SEQ ID NO. 2, SEQ ID NO. 3, SEQ ID NO. 4 and SEQ ID NO. 5.

Preferably, the degree of homology is about 85%, more preferably about 90%, even more preferably about 95%, and most preferably about 99%.

For purposes of the present invention, sequence comparisons are carried out using a Smith-Waterman sequence alignment algorithm (see e.g. Waterman, M.S. Introduction to Computational Biology: Maps, sequences and genomes. Chapman & Hall. London: 1995. ISBN 0-412-99391-0, or at http://www-hto.usc.edu/software/segaln/index.html). The locals program, version 1.16, is used with following parameters: match: 1, mismatch penalty: 0.33, open-gap penalty: 2, extended-gap penalty: 2.

A subsequence is a nucleic acid sequence encompassed by SEQ ID NO. 1, SEQ ID NO. 2, SEQ ID NO. 3, SEQ ID NO. 4 and SEQ ID NO. 5, except that one or more nucleotides from the 5' and/or 3' end have been deleted and which retains part of or the complete promoter activity. For instance, about 50 or about 100 nucleotides may be deleted from the 5' and/or 3' end.

An allelic variant denotes any of two or more alternative forms of a gene occupying the same chomosomal locus. Allelic variation arises naturally through mutation, and may result in polymorphism within populations.

In another embodiment, the present invention relates to isolated nucleic acid sequences which hybridize under conditions of stringency with (i) a sequence selected from the group consisting of SEQ ID NO. 1, SEQ ID NO. 2, SEQ ID NO. 3, SEQ ID NO. 4 and SEQ ID NO. 5; (ii) a subsequence of (i) of at least 100 nucleotides, or (iii) a complementary strand of (i) or (ii).

The subsequence of SEQ ID NO. 1, SEQ ID NO. 2, SEQ ID NO. 3, SEQ ID NO. 4 and SEQ ID NO. 5 may be at least 100 nucleotides or preferably at least 200 nucleotides.

Substantially similar nucleic acid fragments may be characterized by their ability to hybridize to each other under conditions of stringency known by those skilled in the art (Hames and Higgins, Eds. (1985) Nucleic Acid Hybridization, IRL Press, Oxford, UK). Stringency conditions can be adjusted to screen for (highly) similar fragments, such as genes that duplicate functional enzymes from (closely) related organisms. Post hybridization washes determine stringency conditions.
The term "conditions of stringency" is defined herein as a series of washes starting with 6xSSC, 0.5% SDS at room temperature for 15 min, then repeated with 2xSSC, 0.5% SDS at 45°C for 30 min, and then repeated twice with 0.2xSSC, 0.5% SDS at 60°C.
The term "conditions of high stringency" is defined herein as a series of washes starting with 6xSSC, 0.5% SDS at room temperature for 15 min, then repeated with 2xSSC, 0.5% SDS at 45°C for 30 min, and then repeated twice with 0.1xSSC, 0.1% SDS at 65°C.

For purposes of the present invention, hybridization indicates that the nucleic acid sequence hybridizes to a labeled nucleic acid probe corresponding to the nucleic acid sequence shown in SEQ ID NO. 1, SEQ ID NO. 2, SEQ ID NO. 3, SEQ ID NO. 4 and SEQ ID NO. 5, their complementary strands, or subsequences thereof under conditions of stringency, preferably under conditions of high stringency.

The present invention also relates to a nucleic acid construct comprising a nucleic acid sequence encoding a polypeptide operably linked to the nucleic acid sequence described above. It further relates to a recombinant expression vector and a recombinant host cell comprising the nucleic acid construct.

The following Examples will illustrate the present invention but are not intended to limit the present invention in any way.

### Examples

### Example 1

### 1.1. Quantitative PCR expression analysis of the Pc12g09320 gene of P. chrysogenum

In order to confirm the expression pattern and to quantify the high expression level of new gene Pc12g09320 quantitative PCR analysis is performed (ABI7900 HT, Applied Biosystems). Total RNA from mycelia of *P. chrysogenum* is isolated which are grown under various conditions, i.e. under production conditions and collected by filtration on sterile cloth.
The wet mycelium is flash-frozen in liquid nitrogen and ground to a powder using a mortar and pestle. Total RNA is extracted using the TRIZOL^{®} Reagent (Invitrogen) extraction protocol. All procedures are performed according to the manufacturers protocols. After extraction the RNA samples are purified with RNeasy^{®} columns (Qiagen) and an on-column DNase digest is performed using the RNase-Free DNase Set Kit (Qiagen). RNA is resuspended in DEPC-treated, sterile, destilled water and its concentration is measured by spectrophotometry (Ultrospec 3100 pro, Amersham). The quality of the RNA is checked by Bioanalyzer-measurements (Agilent) using the RNA 6000 Nano Assay (Agilent). Subsequent reverse transcription is performed using the High Capacity cDNA Archive Kit (Applied Biosytems). Specific oligonucleotides for the Pc12g09320 gene are designed using the Primer Express software (Applied Biosystems). The forward primer Pc12g09320_U1 (5'-CAAGGCCGACCACAGCTT-3') (SEQ ID NO. 6) and the reverse primer Pc12g09320_L1 (5'-GCCGAGACGGTTGACGAAT-3') (SEQ ID NO. 7) which cover positions 207-224 and 306-288, respectively, of the Pc12g09320 gene sequence are designed. As a control, the known act gene of *P. chrysogenum* is used (Acc. No. AF056975) for the design of the forward primer Pc_actA_U1 (5'-GGTGATGAGGCACAGTCGAAG-3') (SEQ ID NO. 8) and the reverse primer Pc_actA_L1 (5'-AGCTCGTTGTAGAAGGTGTGGTG-3') (SEQ ID NO. 9) which yield a 119-bp amplicon. For PCR reactions the SYBR Green Mastermix (Applied Biosystems) is used. The reaction is performed according to the manufacturers protocol using 20µl reactions which each contained 50ng of template cDNA. A no template control (NTC), with no added template RNA to control for any contaminants in reagents for each template is included. The good efficiency of all primer sets used is validated by standard curves. The results confirm the very strong and constitutive gene expression levels which are also detected by full genome microarray analysis. In particular this permanent and constitutive expression level is detected under production conditions.

### 1.2. Cloning and characterization of the Pc12g09320 promoter region

To create a suitable recipient reporter gene vector the plasmid pGen4.5 is generated in two steps. In the first step a 607 bp terminator region of the *Penicillium chrysogenum* γ-actin gene (Accession No. AF056975) is amplified by PCR with the proof reading, Pwo DNA polymerase (Roche), digested with *Not*I and *Bam*HI and cloned in a pBluescript II KS+ Vector from Stratagene. In the next step a 1812 bp fragment including the coding region of the *E. coli uidA* gene is amplified by PCR with the proof reading, Pwo DNA polymerase (Roche), digested with *Nar*I and *Pst*I and cloned in the pBluescript II KS+ Vector containing the γ-actin terminator region.
On the basis of the Penicillium chrysogenum genome the putative promoter region of the gene Pc12g09320 is evaluated assuming that the coding region of the gene Pc12g09310 upstream of Pc12g09320 delimits its putative promoter region.
The Pc12g09320 promotor region is cloned using the following oligonucleotides as primers: Pc12g09320-for (5'-GTCAAAGCTTGTGTACTTACCAATGGC-3') (SEQ ID NO. 10) and Pc12g09320-rev (5'-AGTCATGCATGCTGAATGAAGGCGGGAGA-3') (SEQ ID NO. 11).
The 351 bp DNA fragment obtained by PCR with the proof reading, Pwo DNA polymerase (Roche) is digested with *Hind*III and Sphl and subcloned in the *Hind*III and *Sph*I digested plasmid pGen4.5 containing the *E.coli uidA* gene and the *Penicillium chrysogenum act* terminator region and generating the plasmid *pGen4.24.* The *uidA* gene of *E*. *coli* is fused translationally with the Pc12g09320 promotor region. This is carried out designing the *Sph*I site such that it is part of the ATG start codon which codes for the initiator methionine of the *uidA* gene.
The sequencing of the amplified promoter region as well as the fusion region between p-Pc12g09320 and the *uidA* gene confirm the correctness of the promoter sequence as well as the exact arrangement of the latter reporter gene in the correct reading frame.

### 1.3. Expression of the uidA gene of E. coli in P. chrysogenum driven by the P-Pc12g09320

For cotransformation of pGen4.24 into P. chrysogenum strain Q176 the plasmid pBC1003 carrying the Tn5-phleomycin resistance gene under the control of the isopenicillin-N-synthetase promoter from *P. chrysogenum* is used as the selection marker (2). Protoplasts are transformed according to Cantoral *et al.* (1), and transformants are selected on minimal medium containing NaNO₃ and glucose as the nitrogen and carbon sources, respectively and 30 µg/ml phleomycin (Sigma). Screening of positive clones is performed by UIDA-Plate assay on minimal medium containing NaNO₃ and glucose as the nitrogen and carbon sources and 50µg/ml of the cyclohexylammonium salt of 5-bromo-4-chloro-3-indolyl-β-D-glucuronic acid (Biosynth) as substrat for the UIDA protein. Positive clones can be identified through their ability to generate biue coioured colonies after 2-5 days incubation at 25°C. To obtain homokaryotic transformants, colonies from single homokaryotic spores are picked, and genomic integration of the expression construct is verified by Southern analysis. Four independent transformants transformants carrying a copy of *pGen 4.24* integrated ectopically are selected for growth tests, Northern blot and qPCR analysis.
Southern analysis confirms the presence of the plasmid *pGen4.24.* The presence of the UIDA transcript can be determined by Northern Blot analysis and the relative amount by qPCR-analysis.
UIDA-Plate assay as well as Northern and qPCR analysis show clearly that the transformed P-Pc12g09320-uidA construct expresses the heterologous bacterial β-glucuronidase gene.

### 1.4. Expression of the uidA gene in P. chrysogenum under control of the Pc12g09320 promoter

The uidA gene of E. coli is fused with the Pc12g09320 promoter as described in Section 1.2. of Example 1. The heterologous expression of the uidA gene is characterized by quantitative PCR (7900HT, Applied Biosystems). Specific oligonucleotides for the *uidA* gene are designed using the Primer Express software (Applied Biosystems). The forward primer Ec_uidA_U1 (5'-TTCATGCCAGTCCAGCGTT-3') (SEQ ID NO. 12) and the reverse primer Ec_uidA_L1 (5'- CGACCGCAAACCGAAGTC-3') (SEQ ID NO. 13) which yield a 56-bp product. The processes of RNA isolation and purification as well as the cDNA synthesis and the subsequents quantitative PCR analysis using the act gene as control are carried out as described in Section 1.1. of Example 1. Four independent *P. chrysogenum* strains which harbor the *uidA* gene under control of the Pc12g09320 promoter are analyzed by qPCR. In all of these strains a very high level of *uidA* mRNA is detected. This result confirms that the Pc12g09320-promoter-uidA construct expresses the heterologous *uidA* gene as well as the endogenous Pc12g09320 gene. This result is in accordance with the strong UIDA enzyme activity which is observed on chromogenic plate medium (see Section 1.3., Example 1).

### Example 2

### 2.1. Quantitative PCR expression analysis of the Pc12g10000 gene of P. chrysogenum

Quantitative PCR analysis is performed to confirm and quantify the high expression level of new gene Pc12g09320 which is detected by full genome microarray analysis. qPCR analysis is performed as described in Section 1.1. of Example 1. Total RNA is isolated from mycelia of *P. chrysogenum* which are grown under various conditions, i.e. under production conditions. Subsequent reverse transcription is performed to generate cDNA which serves as template in qPCR analysis (see Section 1.1. of Example 1). Specific oligonucleotides for the Pc12g10000 gene are designed using the Primer Express software (Applied Biosystems). The forward primer Pc12g10000_U1 (5'-AACAACATTGCGCTCGATGA-3') (SEQ ID NO. 14) and the reverse primer Pc12g10000_L1 (5'-CCTTGGAAACCGTGTTCGTT-3') (SEQ ID NO. 15) which cover positions 100-119 and 199-180, respectively, of the Pc12g10000 gene sequence are designed. The results confirm the very strong and constitutive gene expression levels which are also detected by full genome microarray analysis. The analysis confirms the permanent and constitutive expression level of Pc12g09320 under production conditions.

### 2.2. Cloning and characterization of the Pc12g10000 promoter region

The Pc12g10000 promotor region is cloned using the following oligonucleotides as primers: Pc12g10000-for (5'-GTCAAAGCTTCCGGCTCGGATCTCGTC-3') (SEQ ID NO. 16) and Pc12g10000-rev (5'-GAGGGCATGCTGACTTGTTCACTTCAAGG-3') (SEQ ID NO. 17).
The 764 bp DNA fragment obtained by PCR with the proof reading, Pwo DNA polymerase (Roche) is digested with *Hind*III and *Sph*I and subcloned in the *Hind*III and *Sph*I digested plasmid pGen4.5 containing the *E.coli uidA* gene and the *Penicillium chrysogenum act* terminator region and generating the plasmid *pGen4.25* (see section 1.2., Example 1). The *uidA* gene of *E. coli* is fused translationally with the Pc12g10000 promotor region. This is carried out designing the *Sph*I site such that it is part of the ATG start codon which codes for the initiator methionine of the *uidA* gene.
The sequencing of the amplified promoter region as well as the fusion region between p-Pc12g10000 and the *uidA* gene confirm the correctness of the promoter sequence as well as the exact arrangement of the latter reporter gene in the correct reading frame.

### 2.3. Expression of the uidA gene of E. coli in P. chrysogenum driven by the P-Pc12g10000.

pGen4.25 is transformed and the transformation event verified by the same procedure as described in section 1.3., Example 1.
Southern analysis confirms the presence of the plasmid *pGen4.25.* The presence of the UIDA transcript can be determined by Northern Blot analysis and the relative amount by qPCR-analysis. UIDA-Plate assay as well as Northern and qPCR analysis show clearly that the transformed P-Pc12g10000-*uidA* construct expresses the heterologous bacterial β-glucuronidase gene.

### 2.4. Expression of the uidA gene in P. chrysogenum under control of the Pc12g10000 promoter

The uidA gene of E. coli is fused with the Pc12g09320 promoter as described in Section 2.2. of Example 2. The heterologous expression of the *uidA* gene is characterized by quantitative PCR (7900HT, Applied Biosystems). Specific oligonucleotides for the *uidA* gene are designed as described in Section 1.4. of Example 1. The processes of RNA isolation and purification as well as the cDNA synthesis and the subsequents quantitative PCR analysis using the act gene as control are carried out as described in Section 1.1. of Example 1. Three independent *P. chrysogenum* strains which harbor the *uidA* gene under control of the Pc12g10000 promoter are analyzed by qPCR. In all of these strains a very high level of *uidA* mRNA is detected. This result confirms that the 12g10000-promoter-*uidA* construct expresses the heterologous *uidA* gene as well as the endogenous Pc12g09320 gene. This result is in accordance with the strong UIDA enzyme activity which is observed on chromogenic plate medium (see Section 2.3., Example 2).

### Example 3

### 3.1. Quantitative PCR expression analysis of the Pc16g00660 gene of P. chrysogenum

Quantitative PCR analysis is performed to confirm and quantify the high expression level of new gene Pc16g00660 which is detected by full genome microarray analysis. qPCR analysis is performed as described in Section 1.1. of Example 1. Total RNA is isolated from mycelia of *P. chrysogenum* which are grown under various conditions, i.e. under production conditions. Subsequent reverse transcription is performed to generate cDNA which served as template in qPCR analysis (see Section 1.1. of Example 1). Specific oligonucleotides for the Pc16g00660 gene are designed using the Primer Express software (Applied Biosystems). The forward primer Pc16g00660_U1 (5'-TGAAGTTCGACGAGGACTGATG-3') (SEQ ID NO. 18) and the reverse primer Pc16g00660_L1 (5'-CGCTTGAGACGTCAGGTTGTT-3') (SEQ ID NO. 19) which cover positions 1122-1143 and 1221-1201, respectively, of the Pc16g00660 gene sequence are designed. The pPCR analysis confirms the very strong and constitutive gene expression levels which are also detected by full genome microarray analysis. The analysis confirms the permanent and constitutive expression level of Pc16g00660 under production conditions.

### 3.2. Cloning and characterization of the Pc16g00660 promoter region

The Pc16g00660 promotor region is cloned using the following oligonucleotides as primers: Pc16g00660-for (5'-GTCAAAGCTTGATATGTGGAGCCTGCG-3') (SEQ ID NO. 20) and Pc16g00660-rev (5'-TCATGCATGCTGGCGGTTCTGGAATCCAG-3') (SEQ ID NO. 21).
The 1498 bp DNA fragment obtained by PCR with the proof reading, Pwo DNA polymerase (Roche) is digested with *Hind*III and *Sph*I and subcloned in the *Hind*III and *Sph*I digested plasmid pGen4.5 containing the *E.coli uidA* gene and the *Penicillium chrysogenum act* terminator region and generating the plasmid *pGen4.26* (see section 1.2., Example 1). The *uidA* gene of *E. coli* is fused translationally with the Pc16g00660 promotor region. This is carried out designing the *Sph*I site such that it is part of the ATG start codon which codes for the initiator methionine of the *uidA* gene.
The sequencing of the amplified promoter region as well as the fusion region between p-Pc16g00660 and the *uidA* gene confirm the correctness of the promoter sequence as well as the exact arrangement of the latter reporter gene in the correct reading frame.

### 3.3. Expression of the uidA gene of E. coli in P. chrysogenum driven by the P-Pc16g00660.

*pGen4.26* is transformed and the transformation event verified by the same procedure as described in section 1.3., Example 1.
Southern analysis confirms the presence of the plasmid *pGen4.26.* The presence of the UIDA transcript can be determined by Northern Blot analysis and the relative amount by qPCR-analysis. UIDA-Plate assay as well as Northern and qPCR analysis show clearly that the transformed P-Pc16g00660-*uidA* construct expresses the heterologous bacterial β-glucuronidase gene.

### 3.4. Expression of the uidA gene in P. chrysogenum under control of the Pc16g00660 promoter

The *uidA* gene of *E. coli* is fused with the Pc16g00660 promoter as described in Section 3.2. of Example 3. The heterologous expression of the *uidA* gene is characterized by quantitative PCR (7900HT, Applied Biosystems). Specific oligonucleotides for the *uidA* gene are designed as described in Section 1.4. of Example 1. The processes of RNA isolation and purification as well as the cDNA synthesis and the subsequent quantitative PCR analysis using the act gene as control are carried out as described in Section 1.1. of Example 1. Four independent *P. chrysogenum* strains which harbor the *uidA* gene under control of the Pc16g00660 promoter are analyzed by qPCR. In all of these strains a very high level of *uidA* mRNA is detected. This result confirms that the Pc16g00660-promoter-*uidA* construct expresses the heterologous *uidA* gene as well as the endogenous Pc16g00660 gene. This result is in accordance with the strong UIDA enzyme activity which is observed on chromogenic plate medium (see Section 3.3., Example 3).

### Example 4

### 4.1. Quantitative PCR expression analysis of the Pc21g04830 gene of P. chrysogenum

Quantitative PCR analysis is performed to confirm and quantify the high expression level of new gene Pc21g04830 which is detected by full genome microarray analysis. qPCR analysis is performed as described in Section 1.1. of Example 1. Total RNA is isolated from mycelia of *P. chrysogenum* which are grown under various conditions, i.e. under production conditions. Subsequent reverse transcription is performed to generate cDNA which served as template in qPCR analysis (see Section 1.1 of Example 1). Specific oligonucleotides for the Pc21g04830 gene are designed using the Primer Express software (Applied Biosystems). The forward primer Pc21g04830_U1 (5'-TTGTCCTGGTCTTTCCCCATT-3') (SEQ ID NO. 22) and the reverse primer Pc21g04830_L1 (5'-CCAGGCCTACCGTACCATTG-3') (SEQ ID NO. 23) which cover positions 490-510 and 589-570, respectively, of the Pc21g04830 gene sequence are designed. The pPCR analysis confirmed the very strong and constitutive gene expression levels which are also detected by full genome microarray analysis. The analysis confirms the permanent and constitutive expression level of Pc21g04830 under production conditions.

### 4.2. Cloning and characterization of the Pc21g04830 promoter region

The Pc21g04830 promotor region is cloned using the following oligonucleotides as primers: Pc21g04830-for (5'-GTCAAAGCTTGAATTCCATCGCCGGGTCGCC-3') (SEQ ID NO. 24) and Pc21g04830-rev (5'-TCATGCATGCTCGAGGAAGGGAGGAGAGG-3') (SEQ ID NO. 25).
The 1363 bp DNA fragment obtained by PCR with the proof reading, Pwo DNA polymerase (Roche) is digested with *Hind*III and *Sph*I and subcloned in the *Hind*III and *Sph*I digested plasmid pGen4.5 containing the *E.coli uidA* gene and the *Penicillium chrysogenum act* terminator region and generating the plasmid *pGen4.27* (see section 1.2., Example 1). The *uidA* gene of *E*. *coli* is fused translationally with the Pc21 g04830 promotor region. This is carried out designing the *Sph*I site such that it is part of the ATG start codon which codes for the initiator methionine of the *uidA* gene.
The sequencing of the amplified promoter region as well as the fusion region between p-Pc21g04830 and the *uidA* gene confirm the correctness of the promoter sequence as well as the exact arrangement of the latter reporter gene in the correct reading frame.

### 4.3. Expression of the uidA gene of E. coli in P. chrysogenum driven by the P-Pc21 g04830.

*pGen4.27* is transformed and the transformation event verified by the same procedure as described in section 1.3., Example 1.
Southern analysis confirms the presence of the plasmid *pGen4.27.* The presence of the UIDA transcript can be determined by Northern Blot analysis and the relative amount by qPCR-analysis. UIDA-Plate assay as well as Northern and qPCR analysis show clearly that the transformed P-Pc21g04830-*uidA* construct expresses the heterologous bacterial β-glucuronidase gene.

### 4.4. Expression of the uidA gene in P. chrysogenum under control of the Pc21g04830 promoter

The *uidA* gene of *E. coli* is fused with the Pc21 g04830 promoter as described in Section 4.2. of Example 4. The heterologous expression of the *uidA* gene is characterized by quantitative PCR (7900HT, Applied Biosystems). Specific oligonucleotides for the *uidA* gene are designed as described in Section 1.4. of Example 1. The processes of RNA isolation and purification as well as the cDNA synthesis and the subsequent quantitative PCR analysis using the act gene as control are carried out as described in Section 1.1. of Example 1. Four independent *P. chrysogenum* strains which harbor the *uidA* gene under control of the Pc21g04830 promoter are analyzed by qPCR. In all of these strains a very high level of *uidA* mRNA is detected. This result confirms that the Pc21g04830-promoter-*uidA* construct expresses the heterologous *uidA* gene as well as the endogenous Pc16g00660 gene. This result is in accordance with the strong UIDA enzyme activity which is observed on chromogenic plate medium (see Section 3.3., Example 3).

### Example 5

### 5.1. Quantitative PCR expression analysis of the Pc21g20300 gene of P. chrysogenum

Quantitative PCR analysis is performed to confirm and quantify the high expression level of new gene Pc21g20300 which is detected by full genome microarray analysis. qPCR analysis is performed as described in Section 1.1. of Example 1. Total RNA is isolated from mycelia of *P. chrysogenum* which are grown under various conditions, i.e. under production conditions. Subsequent reverse transcription is performed to generate cDNA which served as template in qPCR analysis (see Section 1.1. of Example 1). Specific oligonucleotides for the Pc21g20300 gene are designed using the Primer Express software (Applied Biosystems). The forward primer Pc21g20300_U1 (5'-CTGCAGAGCGATGGTTGCT-3') (SEQ ID NO. 26) and the reverse primer Pc21g20300_L1 (5'-TTGGAGACAGAGACTTGGTCCTT-3') (SEQ ID NO. 27) which cover positions 103-121 and 230-208, respectively, of the Pc21 g20300 gene sequence are designed. The pPCR analysis confirms the very strong and constitutive gene expression levels which are also detected by full genome microarray analysis. The analysis confirms the permanent and constitutive expression level of Pc21 g20300 under production conditions.

### 5.2. Cloning and characterization of the Pc21g20300 promoter region

The Pc21g20300 promotor region is cloned using the following oligonucleotides as primers: Pc21g20300-for (5'-GTCAGAATTCCCCTTCTGGTGATTG-3') (SEQ ID NO. 28) and Pc21g20300-rev (5'-GCTGATGCATCTTGATGGATTGACT-3') (SEQ ID NO. 29).
The 1359 bp DNA fragment obtained by PCR with the proof reading, Pwo DNA polymerase (Roche) is digested with *Eco*RI and *Nsi*I and subcloned in the *Eco*RI and *Nsi*I digested plasmid pGen4.5 containing the *E.coli uidA* gene and the *Penicillium chrysogenum act* terminator region and generating the plasmid *pGen4.30* (see section 1.2., Example 1). The *uidA* gene of *E. coli* is fused translationally with the Pc21 g20300 promotor region. This is carried out designing the *Nsi*I site such that it is part of the ATG start codon which codes for the initiator methionine of the *uidA* gene.
The sequencing of the amplified promoter region as well as the fusion region between p-Pc21g20300 and the *uidA* gene confirm the correctness of the promoter sequence as well as the exact arrangement of the latter reporter gene in the correct reading frame.

### 5.3. Expression of the uidA gene of E. coli in P. chrysogenum driven by the P-Pc21g20300.

*pGen4.30* is transformed and the transformation event verified with the same procedure described in section 1.3., Example 1.
Southern analysis confirms the presence of the plasmid *pGen4.30.* The presence of the UIDA transcript can be determined by Northern Blot analysis and the relative amount by qPCR-analysis. UIDA-Plate assay as well as Northern and qPCR analysis show clearly that the transformed P-Pc21g20300-*uidA* construct expresses the heterologous bacterial β-glucuronidase gene.

### 5.4. Expression of the uidA gene in P. chrysogenum under control of the Pc21g20300 promoter

The uidA gene of E. coli is fused with the Pc21 g20300 promoter as described in Section 5.2. of Example 5. The heterologous expression of the *uidA* gene is characterized by quantitative PCR (7900HT, Applied Biosystems). Specific oligonucleotides for the *uidA* gene are designed as described in Section 1.4. of Example 1. The processes of RNA isolation and purification as well as the cDNA synthesis and the subsequent quantitative PCR analysis using the act gene as control are carried out as described in Section 1.1. of Example 1. Three independent *P. chrysogenum* strains which harbor the *uidA* gene under control of the Pc21g20300 promoter are analyzed by qPCR. In all of these strains a very high level of *uidA* mRNA is detected. This result confirms that the Pc21g20300-promoter-*uidA* construct expresses the heterologous *uidA* gene as well as the endogenous Pc21 g20300 gene. This result is in accordance with the strong UIDA enzyme activity which is observed on chromogenic plate medium (see Section 5..3, Example 3).

### Cited References

1 Cantoral, I. et al. "High frequency transformation of Penicillium chrysogenum" J. Biotechnol. 5 (1987) pp. 494-497.
2 Kolar, M. et al. "Transformation of Penicillium chrysogenum using dominant selection markers and expression of an Escherichia coli lacZ fusion gene" Gene 62 (1) (1988) pp. 127-134.
3 Zadra, I. et al. "xylP promoter-based expression system and its use for antisense downregulation of the Penicillium chrysogenum nitrogen regulator NRE" Appl. Environ. Microbiol. 66(11) (2000) pp. 4810-4816.
4 Bautista, L. F. et al. "Antisense Silencing of the creA Gene in Aspergillus nidulans" Appl. Environ. Microbiol. 66(10) (2000) pp. 4579-4581.
5 Wang, T. H. et al. "Antisense inhibition of xylitol dehydrogenase gene, xdh1 from Trichoderma reesei" Lett. Appl. Microbiol. 40 (2005) pp. 424-429.
6 Nakayashiki, H. et al. "RNA silencing as a tool for exploring gene function in ascomycete fungi" Fungal Genetics and Biology 42 (2005) pp. 275-283.
7 Kadotani, N., et al. "RNA-silencing in the Phytopathogenic Fungus Magnaporthe oryzae" Mol. Plant-Microbiol. Interactions 16(9) (2003) pp. 769-776.
8 Mouyna, I., et al. "Gene silencing with RNA interference in the human pathogenic fungus Aspergillus fumigatus" FEMS Microbiol. Lett. 237 (2004) pp. 317-324.
9 Finklstein, Ball (eds.), Applied Molecular Genetics of Filamentous fungi (ibid.) Biotechnology of Filamentous fungi, Butterworth-Heinemann, Boston (1992).
10 Hames and Higgins (eds.) Nucleic Acid Hybridization, IRL Press, Oxford, UK (1985).

## Claims

1. A method for producing a polypeptide, comprising the steps of:
(a) cultivating a fungal host cell in a medium conductive for the production of the polypeptide, wherein the fungal host cell comprises a first nucleic acid sequence encoding the polypeptide operably linked to a second nucleic acid sequence comprising a promoter sequence selected from the group consisting of SEQ ID NO. 1, SEQ ID NO. 2, SEQ ID NO. 3, SEQ iD NO. 4, SEQ ID NO. 5, and subsequences thereof, and mutant, hybrid, and tandem promoters thereof; and
(b) isolating the polypeptide from the culture medium.

2. A method according to claim 1, wherein the fungal host cell in step a) is a filamentous fungal host cell.

3. A method according to claim 1 or 2, wherein the fungal host cell in step a) is Penicillium chrysogenum or Acremonium chrysogenum or Aspergillus terreus or Penicillium citrinum.

4. Isolated nucleic acid sequence comprising a nucleic acid sequence selected from the group consisting of SEQ ID NO. 1, SEQ ID NO. 2, SEQ ID NO. 3, SEQ ID NO. 4, SEQ ID NO. 5 and subsequences thereof.

5. Isolated nucleic acid sequence, selected from the group consisting of
(a) a nucleic acid sequence having at least 80% homology with a sequence selected from the group consisting of SEQ ID NO. 1, SEQ ID NO. 2, SEQ ID NO. 3, SEQ ID NO. 4 and SEQ ID NO. 5;
(b) a nucleic acid sequence which hybridizes under conditions of stringency with (i) a sequence selected from the group consisting of SEQ ID NO. 1, SEQ ID NO. 2, SEQ ID NO. 3, SEQ ID NO. 4 and SEQ ID NO. 5; (ii) a subsequence of (i) of at least 100 nucleotides, or (iii) a complementary strand of (i) or (ii);
(c) a nucleic acid sequence comprising a substitution, deletion, and/or insertion of one or more nucleotides;
(d) an allelic variant of (a), (b), or (c); and
(e) a subsequence of (a), (b), (c), or (d).

6. A nucleic acid construct comprising a nucleic acid sequence encoding a polypeptide operably linked to the nucleic acid sequence of claim 4 or 5.

7. A recombinant expression vector comprising the nucleic acid construct of claim 6.

8. A recombinant host cell comprising the nucleic acid construct of claim 6.


### Annex to the application documents - subsequently filed sequences listing

SEQUENCE LISTING

<110>  Sandoz AG

<120>  Promoter sequences

<130>  IB/G-34757P1

<140>  EP05028139.3
<141>  2005-12-22

<160>  29

<170>  PatentIn version 3.3

<210>  1
<211>  331
<212>  DNA
<213>  Penicillium chrysogenum

<400>  1
ttgtgtactt accaatggct atctctcata aactatcgaa gcctggtcct ttcttaaagc      60

cagccacata aagctaccta ggtagcttca gcccgtgatc aaacccaatg ctaccctgca     120

acatacacta ttttgactat tgacttatct cttgtaatct ggacttggtc ttgcctactc     180

tgggggtaca tcgtacaaac ttgctatctt ttactttttt tgcttttaag atctcaatgt     240

ctccttttta atcttccttc tcactcacac acctcttaat attcccacac cttttatcgt     300

ctattcttct gcggtctccc gccttcattc a                                    331

<210>  2
<211>  744
<212>  DNA
<213>  Penicillium chrysogenum

<400>  2
ccggctcgga tctcgtcata gagcaagggg cattttgata cggatttgcg gctcttggag      60

atagttggta agatatcatc ggtgacattg tgtttatatc tcactccttt catcgttaaa     120

gaacacactg taattgatat tgtggtgatc gagagtactt gaaatataaa gacatttttc     180

tgaacaggtg attataaata tgcgcaacaa caccggggac tgtcgaccat cgaatatcat     240

attcaaattt tgcggctcaa accaaaaaag aatatactgt atgttctaag gttgtagtct     300

atcctccgca gaaccagaac tacgagctac gctgccccac cttgtccgaa tgtagcatag     360

taaccatgca gatccttgcc aaatccttca tccctgcagc cccctgacca gatacctaag     420

caggtaacct tagaagacaa gagaaccagt gattacaaat ccgacttcgg tgtaagcatt     480

ggaatttctt ttgctgaggg gtagaatatc tcgtataggt aggtaagaca acgttggaag     540

gggctcactg attacaaatc ggacctcatt tccaagattg cctgaggatt tcttctgctg     600

ggggaggggg atatcgtgta tataaggcct catctacttc cctctacatg ataataataa     660

taccgaatca atcgatccat cattgttgac tctatttcta cggcctacca gtgttaaacc     720

actcaccttg aagtgaacaa gtca                                            744

<210>  3

<211>  1491
<212>  DNA
<213>  Penicillium chrysogenum

<400>  3
cttgatatgt ggagcctgcg tagattgaca tataatctga caatgcaaga atgcagacgt      60

gcatggtcac gcgatgggat ggatatcgac ccaaaacaag gctaagccaa ttcggctagt     120

cgcagctcag cagttatcgt ccaattaaat ggtcaattgg acgtgcaagg atatggaggt     180

caatgcagag caagtgggca aggttgtctg ggaatgtgga gtcatgcgaa gcggtgaaca     240

tctgccaaat ctggggttaa tgagttttga ccacgccaaa gtgaaagtga gcaagtcgac     300

gagattgatg catctcggtt ggggcatgaa tcacccgtga aggtcgagcg catcactcct     360

gccagcccat cgagggacgc agtggacgag gtgagatcgg gtggtcgttc acatcaaaac     420

acgtgggttc ccacagacga taagctgggt atccaccaag actcgagcga tagatacaat     480

cagtacgcgg taaaagagac atgcggtttg tactgtggaa tatcgatata ttgtaattgg     540

aaggaagagc aatggggagg ctgaatgaga ccaacagagg ccaatagcct ccaacaagga     600

cactcagctt gatagtcacg aggttcaaag gattcccggg acaccgacgg tccaagaagt     660

ccggaaacag ttttgtaaaa aacgagtcgg gtaattttga atggcaacat cacatggggc     720

caatcacacc atcgatggat gtctcatttc cacaagtggc aatttcacct tgagcctaat     780

cagaaagccc cgcatctttg accaggtgca gttgcctgtg attgggtccc aggccatgtg     840

tgagatatca aagtccagct atctcacctc ggttatcagt cattcttcaa tgtgataggc     900

tggatgagat ggccaagaga cagggttaca atggaagtta ccagccaagc ctcccgacgc     960

ggggagaaac tcggccaaac cgtgggccaa gatataccaa gacgcactaa cccgacacta    1020

agtaacacca agtggatatc aatcccaggg gtctcagccc acagtagctc attgattggt    1080

tgatccaaat tcagacatcc attagcctct cgggatagtt atcttttact tttccaacaa    1140

agcgcgactt ccaaggtttt gcttatcgac ggggaaagtg gtcacggccg gatgaggccg    1200

aagctcactt ccaaggttaa tctcgtaaaa gcactaaaag cactaagacg cagggtcttc    1260

gccggctatt gacggttttt gccaagtccg ccgtccgcca cgtcccaatc cagacattgc    1320

cccgggattc tggaatgaga gaagcgagac tcttggaggc actaaacagg tacgctaaat    1380

cacgccatgg accatataat tgcttcacct cgccctccca caagttttct tttgcttttg    1440

ctttcctgct gtgtgtttca tctctcacat ctctggattc cagaaccgcc a             1491

<210>  4
<211>  1349
<212>  DNA
<213>  Penicillium chrysogenum

<400>  4
tccatcgccg ggtcgcctct tgtctttttg atttacctag ataggtatga tgtgatattt      60

gcaacatagg tgacgtcatt ttagtttact ggaacatcgc cagcaaagtg ggattataga     120

gatttgaaac gagagggaat gttgatccat ttgaagggta tcatcgtgat cgataatggc     180

aggtggttga caccgggttg gacagcatgt tcaacgctac gcggttgatg aggctcaaag 240
ggaggaccct gttacctgca cagggtgaca ggggcaaagt ctatgtggat acaccaaagt 300
atcagactat gattctacag ttctatagca atatagagct atagagctaa aaggagaagt 360
atatccgaga cataagttgc aacacagaat agaatgccct tagtacatta gatcggagaa 420
gatgtttcag attcagccaa gacaagaaaa gaaaaaaaag atgtttttct aaggggaaat 480
aaaagagagg tgagttaagt agaagattta gttccacatg taaccgtccc cccagttggg 540
ccgactacca ccgttgcact caagggggtc attgagtatg tatgggtaca ttgggaatgt 600
gctcggtatt atattcttgt gttcttatat tctaaatgat ttggagtatt attctatcta 660
aggaatatat tagatctgga ctgtatgact atcaactatt aacactatta catacactat 720
taacctacgt aggttctata aagtaaagta atacatatac aacagcacat gtaactccac 780
tccacccatt actacaggtg tggtgtatgt tgtatatggg atataaactg tatatgaatg 840
gtatggtatg agaatggcct ttttatgaac tgttaatgac tcttctaaag tgactgtaca 900
atagccaaaa gcaataacct ccgtagattt atttcctgtc acctgcacaa catagacata 960
aattaccgtt acagtaccat acccatgcac attgcgacat cgcaagggct tttcccccag 1020
ttgcacccgc ggtgacactg tcaactcaag aatgggccaa tgttgatcca tgcttagggt 1080
caacaccctt cttttccttc tcttctcttc ttctctctcc ttttcacttt ctccgggtgg 1140
ggaattactg cttcactccc accgatccac tcaaaacaca actccagtgg gagtgtcaca 1200
tcggcttgca cccccccccc acccccccaa accttagctt ataacttcag attgttcttc 1260
ttttatctca tctgctccat gatcaatcat atttcaacat agtgaacttc gatcccggtt 1320
cgcgacctcc cctctcctcc cttcctcga 1349

<210> 5
<211> 1442
<212> DNA
<213> Penicillium chrysogenum

<400> 5
ttccccttct ggtgattgac cccaacccca gggcccaccc ccgctaaatg tctcccttgg 60
gtggtggacc cgtttcccag cccgttcttc tcctacgtcg ccagtgcttt ttttggtttg 120
aaaaacaaca gtcgattcgg aagtagcatc ttgactctat tgccactcgg agtggtcacc 180
atatgcaggt acaatattta tcgagacact cggaggatgc taacctttcg gtaacttgct 240
ctgtggtaag gccgggagat atcaagtgac cattaagctc aatgagactc tgcaagcgcc 300
cggggtatct ttgaggcctt tatttgaccc atcagagcta ctatgtacaa cgttgtacct 360
ccataagccc tgtattggat aataagttag ctcatagggt tatggtctcc tgagtttggg 420
ccctttgcag aacctcttat tatttgagga ggcaaattat ggcgctgtgt gtgaccccaa 480
gcaatttcaa tcaagatata tttataccta gttatgtcgt attattgtca ttattgtcat 540
tattgtcatt tcatattaat tagacaatgc gtcggaccaa gtcccaatgt ggtatagttc 600
gatgctacgc tctctcgggc cctaccgggg tccaatcttc cccggatttt cctcaaaaca 660

gagactatgg ggtttgccac tttatgatgc ttgtcaataa ttctgtcata cattcatgca 720

attgttttgg tctgcatatc ttatttgatt gctttgtagc aaacttcaag gttctagccc 780

gtgggaacgc tgcacaaccg ctacatgtac gaagtactgc aacttgtacc gtaccgccat 840

tcaggccgct aagagttcta gcccgacaca ggggaaatta aagtctgtga tgctattcga 900

ccgcatgaca acttacgact aagaagtgta ctaccaacat tgccactggt ccagtctttt 960

cggtggcttg ctcgcttgtc tggtctagtc tcgacttggc gatgcgggca tacgtacgcc 1020

gtacgagaca atagaccaac cccgatcctc gctccaatgg tcaatcggct gcgggaaccc 1080

agaatccggc gtcagccttc aggcttggcc atgcaatgag catccggggt tctaatccgt 1140

ggtaattacg gatctgagtc aaccgcggcc ttggcctact ttcggtggat tgtctctcat 1200

taagcatgcg gagacatgtg atggtcccac gcgccaacct ttgagtgcaa cccttgccca 1260

caagggtttc tccagcgggg aggtggagtg taaaggaccg gcaaagctta taaaaaggcc 1320

atctcccaag cccagacctg gctcttcttc tcaactcatc cgcactcgct atacatactt 1380

gactgtcttt tccggcacgc cgttcataat atcttctatt ttttagtcaa tccatcaaga 1440

tg 1442

<210> 6
<211> 18
<212> DNA
<213> artificial

<220>
<223> forward primer

<400> 6
caaggccgac cacagctt 18

<210> 7
<211> 19
<212> DNA
<213> Artificial

<220>
<223> reverse primer

<400> 7
gccgagacgg ttgacgaat 19

<210> 8
<211> 21
<212> DNA
<213> Artificial

<220>
<223> forward primer

<400> 8
ggtgatgagg cacagtcgaa g 21

<210> 9
<211> 23

<212>  DNA
<213>  Artificial

<220>
<223>  reverse primer

<400>  9
agctcgttgt agaaggtgtg gtg                                              23


<210>  10
<211>  27
<212>  DNA
<213>  Artificial

<220>
<223>  primer

<400>  10
gtcaaagctt gtgtacttac caatggc                                          27


<210>  11
<211>  29
<212>  DNA
<213>  Artificial

<220>
<223>  primer

<400>  11
agtcatgcat gctgaatgaa ggcgggaga                                        29


<210>  12
<211>  19
<212>  DNA
<213>  Artificial

<220>
<223>  forward primer

<400>  12
ttcatgccag tccagcgtt                                                   19


<210>  13
<211>  18
<212>  DNA
<213>  Artificial

<220>
<223>  reverse primer

<400>  13
cgaccgcaaa ccgaagtc                                                    18


<210>  14
<211>  20
<212>  DNA
<213>  Artificial

<220>
<223>  forward primer

<400>  14
aacaacattg cgctcgatga                                                  20

<210>  15
<211>  20
<212>  DNA
<213>  Artificial

<220>
<223>  reverse primer

<400>  15
ccttggaaac cgtgttcgtt                                                    20


<210>  16
<211>  27
<212>  DNA
<213>  Artificial

<220>
<223>  primer

<400>  16
gtcaaagctt ccggctcgga tctcgtc                                            27


<210>  17
<211>  29
<212>  DNA
<213>  Artificial

<220>
<223>  primer

<400>  17
gagggcatgc tgacttgttc acttcaagg                                          29


<210>  18
<211>  22
<212>  DNA
<213>  Artificial

<220>
<223>  forward primer

<400>  18
tgaagttcga cgaggactga tg                                                 22


<210>  19
<211>  21
<212>  DNA
<213>  Artificial

<220>
<223>  reverse primer

<400>  19
cgcttgagac gtcaggttgt t                                                  21


<210>  20
<211>  27
<212>  DNA
<213>  Artificial

<220>

<223>  primer

<400>  20
gtcaaagctt gatatgtgga gcctgcg                                          27


<210>  21
<211>  29
<212>  DNA
<213>  Artificial

<220>
<223>  primer

<400>  21
tcatgcatgc tggcggttct ggaatccag                                        29


<210>  22
<211>  21
<212>  DNA
<213>  Artificial

<220>
<223>  forward primer

<400>  22
ttgtcctggt ctttccccat t                                                21


<210>  23
<211>  20
<212>  DNA
<213>  Artificial

<220>
<223>  reverse primer

<400>  23
ccaggcctac cgtaccattg                                                  20


<210>  24
<211>  31
<212>  DNA
<213>  Artificial

<220>
<223>  primer

<400>  24
gtcaaagctt gaattccatc gccgggtcgc c                                     31


<210>  25
<211>  29
<212>  DNA
<213>  Artificial

<220>
<223>  primer

<400>  25
tcatgcatgc tcgaggaagg gaggagagg                                        29


<210>  26
<211>  19

<212>  DNA
<213>  Artificial

<220>
<223>  forward primer

<400>  26
ctgcagagcg atggttgct                                                    19


<210>  27
<211>  23
<212>  DNA
<213>  Artificial

<220>
<223>  reverse primer

<400>  27
ttggagacag agacttggtc ctt                                               23


<210>  28
<211>  25
<212>  DNA
<213>  Artificial

<220>
<223>  primer

<400>  28
gtcagaattc cccttctggt gattg                                             25


<210>  29
<211>  25
<212>  DNA
<213>  Artificial

<220>
<223>  primer

<400>  29
gctgatgcat cttgatggat tgact                                             25